# EUROPEAN PATENT SPECIFICATION

(11) **EP 1 230 550 B1**
(45) Date of publication and mention of the grant of the patent: **25.01.2006**
(21) Application number: 00978170.9
(22) Date of filing: 15.11.2000
(51) Int. Cl.: G01N 33/543, B01L 3/00

(54) **FLOW MATRIX ASSAY DEVICE WITH MOVABLE SEPARATING MEMBER**
ANALYSEVORRICHTUNG UND IHR GEBRAUCH
DISPOSITIF D'ANALYSE ET SON UTILISATION

(30) Priority: 18.11.1999 SE 9904175
(43) Date of publication of application: 14.08.2002
(73) Proprietor: Pharmacia Diagnostics AB, 751 37 Uppsala (SE)
(72) Inventor: MENDEL-HARTWIG, Ib, S-756 55 Uppsala (SE); BJÖRKMAN, Rune, S-752 36 Uppsala (SE)
(74) Representative: Lindgren, Anders
(86) International application number: PCT/SE2000/002243
(87) International publication number: WO 2001/036974

(56) References cited:
- EP-A1- 0 320 240
- EP-A1- 0 427 534
- WO-A-91/19980
- US-A- 4 959 324
- US-A- 5 260 221

## Description

### Field of the invention

The present invention relates to a novel solid phase assay device for conducting assays, especially immunochromatographic assays, for the determination of analytes in samples, and to methods of using the device.

### Background of the invention

A type of solid phase assay devices comprises a plate-shaped flow matrix of bibulous material, usually a membrane strip, such as of cellulose nitrate or glass fiber, in which liquid can be transported laterally (i.e. in the plane of the strip) by capillary forces in the membrane. The membrane usually has a sample application zone, and a detection zone downstream of the sample application zone. In the detection zone, usually a capturing reagent for the analyte is immobilized. To conduct an assay, the application zone is contacted with the liquid sample to be assayed for the analyte of interest. The device is maintained under conditions sufficient to allow capillary action of liquid to transport the analyte of interest, if present in the sample, through the membrane strip to the detection zone where the analyte is captured. The capillary liquid flow is usually insured by an absorbing pad or the like at the downstream end of the strip. A detection reagent, usually labelled, is then added upstream of the detection zone and interacts with captured analyte in the detection zone, and the amount of captured analyte is measured. Often, the detection reagent is pre-deposited in or on the membrane strip, e.g. in the form of diffusively movable particles containing fluorophoric or chromogenic groups, either upstream of the sample application zone or between the sample application zone and the detection zone.

EP-A-306 336 discloses an assay device of the general type outlined above, wherein the strip of bibulous material is enclosed in a housing. The housing has a first opening for introducing the sample into the device, and second opening for introducing another liquid reagent than the sample into the device, such as a member of the signal producing system used. The device can also include additional means than the two openings for introducing additional assay reagents into the device, e.g. a third opening in the housing or a breakable container with liquid reagent included in the device. The device is said to permit timed reagent additions even though the operator carries out all the steps in rapid succession.

WO 99/36776 discloses an assay method of the general type described above based on the discovery that zonewise migration of desired assay liquids in a predetermined order may be obtained if the liquids are added simultaneously or almost simultaneously to adjacent zones in the strip.

Carrying out an assay with one of the devices described in the two publications mentioned above require, however, a number of steps to be taken by the operator in a short time, and especially the simultaneous addition of liquids according to the method of WO 99/36776 may be difficult or inconvenient for the operator to perform.

US 4,959,324 US 5,260,221 and EP 0 427 534 disclose test devices comprising two bibulous strips for containing buffer and detection reagent, which are separated by a gap that is bridged via a movable element.

### Summary of the invention

An object of the present invention is to provide an assay device for conducting an assay for the determination of an analyte, which device permits simultaneous initiation of flow of sample and at least one other assay liquid.

Another object of the present invention is to provide an assay device which is suitable for performing a sequential assay with a predetermined flow of sample and assay liquids through the device.

Still another object of the present invention is to provide a device which is easy to handle for the operator and requires a minimum of operation steps.

According to the present invention, the above and other objects and advantages are obtained with a test device for conducting an assay for the determination of an analyte in a sample, as defined in appended claim 1.

The term "liquid container" is to be interpreted broadly and basically encompasses any liquid holding element or means capable of receiving and delivering liquid. Thus, the liquid container may be a receptacle or well with the opening facing the flow matrix, wherein the opening is closed or sealed by the above-mentioned liquid-tight element. The liquid container may also be a body capable absorbing and holding a predetermined amount of aqueous liquid, such as, for example, a pad or a sponge body. Usually, such a body is enclosed in a well or other room sealed by the liquid-tight element.

In one embodiment, the liquid containers are mounted adjacent to a face, usually the top face, of the flow matrix, and the separation means comprise a flat liquid-tight element sandwiched between the liquid containers and the flow matrix. Preferably, this liquid-tight element is at least partially removable from the housing and may, for example, be a pull-out film.

In an alternative embodiment, the liquid containers are mounted in a movable relationship with the flow matrix, i.e. in a first position, the liquid containers are separated from the flow matrix, and may be brought to a second position where the containers are in liquid transferring contact with the flow matrix.

### Brief description of the drawings

Fig. 1 is a perspective view of an embodiment of a device according to the present invention.
Fig. 2 is a top view of the lower part of the device in Fig. 1.
Fig. 3 is a partially transparent bottom view of the upper part of the device in Fig. 1.
Fig. 4 is a sectional side view of the device in Fig. 1.
Fig. 5 is an exploded view corresponding to the side view in Fig. 4.
Fig. 6 is an exploded partial view of another embodiment of device according to the present invention.

### Detailed description of the invention

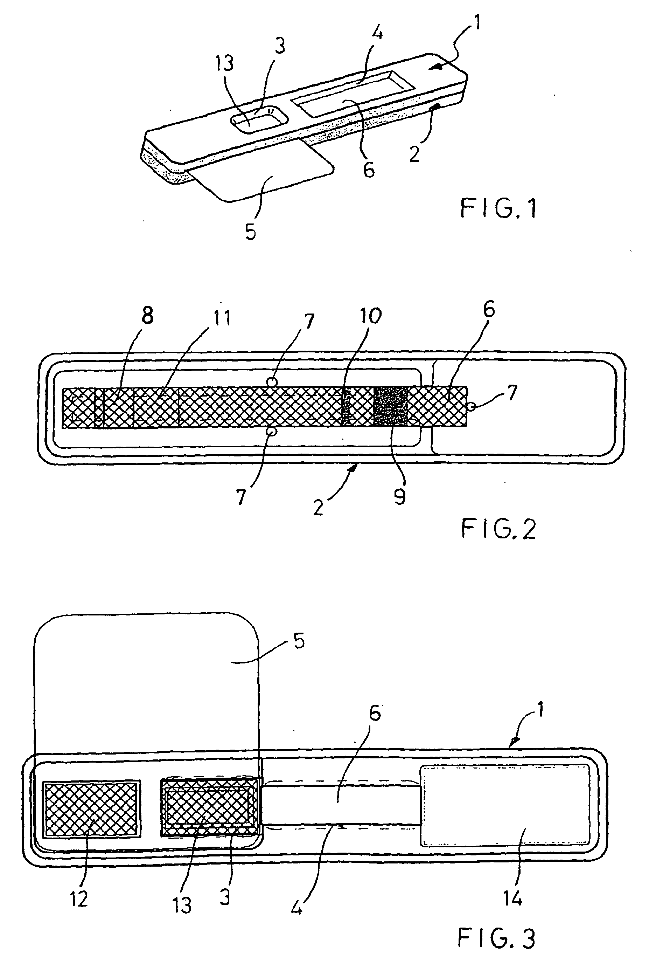

As best shown in Fig. 1, the device illustrated in Figures 1 to 5 comprises an upper housing part I and lower housing part 2 of a material which is inert with respect to the sample and any reagents used in the assays to be conducted with the device, e.g. polystyrene or polypropylene. The upper housing part 1 has a sample well aperture 3 (here conical) and a detection window 4. Also shown in Fig. 1 is a removable separation means 5 to be described below.

With reference now to primarily to Fig. 2, but also to Figs. 3 to 5, the lower housing part 2 has mounted therein a test strip 6 of bibulous material (i.e. a porous material susceptible to traversal of an aqueous medium due to capillary action), e.g. nitrocellulose on a polyester backing. In order to avoid capillary effects along the edges of the strip, the strip 6 is mounted on a ridge (dashed line) in the housing part bottom, the ridge being narrower than the width of the strip. The positioning of the strip 6 in the housing is facilitated by guide means 7. Near the upstream end of the strip 6 (to the left in Fig. 2), a filter piece 8, containing a diffusively movable detection reagent, is placed on the strip. Such a detection reagent may, for example, be a conjugate between a-label particle and a reactant capable of binding to the analyte. Further downstream, and placed below and within the detection window 4, there is a reaction zone 9 on the strip which contains an immobilized reactant capable of binding an analyte to be tested for. In the illustrated case, there is also a calibrator zone 10 containing a predetermined amount of immobilized calibrator substance, for example analyte. Also depicted on the membrane strip 6 is a flow barrier 11, here specifically a piece of a film element, which covers the filter piece 8 and extends towards the opening 3 in the housing part 1. The function of the flow barrier 11 will be described further on.

Turning specifically to Figs. 3 to 5, the upper housing part 1 contains at the upstream end of the membrane strip 6, a pad 12 of liquid absorbing material intended to serve as a container for flow liquid, or buffer. The opening 3 in housing part 1 (Fig. 1) is intended for introducing sample to the membrane 6. In the illustrated case, a filter element 13 (which optionally may consist of two or more separate filters), is provided below the opening 3 for assays where the sample liquid needs to be filtered, e.g. when the sample is whole blood and blood cells are to be separated off. The buffer pad 12 thus forms a buffer liquid container, below referred to as buffer pad, and the room defined by the sample opening 3 and the filter element 13 forms a sample well, or sample container.

At the downstream end of the membrane strip 6, a pad 14 of absorbent material is placed, the purpose of which is to assist in maintaining a capillary flow of assay liquids through the membrane strip 6.

The above-mentioned separation element 5, here a liquid-tight pull-out film, is mounted at the upstream part of the membrane strip 6 to prevent contact between the membrane strip 6 and the bottom parts of the buffer pad 12 and sample filter 13, respectively. The film 5 is arranged to be manually removed by pulling it away from the device to thereby expose the top face of the membrane strip 6 to the buffer pad 12 (except the part of the membrane strip covered by the flow barrier film 11) and the sample filter 13, respectively, such that the membrane strip 6 is brought into simultaneous or close to simultaneous liquid receiving contact with the buffer pad 12 and the filter 13 in the sample well 3. The upper housing part 1 has a recess 15 for the buffer pad 12 designed to press the pad against the pull-out film 5, and thereby against the membrane strip 6 and flow film 11 when the pull-out film 5 is removed. To insure a liquid-tight enclosure of the pad 12 in the recess 15, the pull out film is tightly sealed against the edges of the recess 15, e.g. by welding. While in the illustrated case above, the pull-out film 5 is intended to be removed completely from the device, it is of course, sufficient that the film 5 is withdrawn from the membrane strip 6 to such an extent that the membrane strip surface parts in question are exposed to the sample and buffer liquids, respectively.

An assay for an analyte in a sample may be performed with the device described above as follows.

The device is usually provided ready for use with the buffer pad 12 soaked with buffer solution (flow liquid), with the detection reagent pre-deposited in the filter 8, and with the respective appropriate capture reagents immobilized in the reaction (or detection) zone 9 and the calibration zone 10, respectively. If the analyte to be tested for is, say, an antigen, the detection reagent in the filter 8 may, for example, be an antibody to the antigen coupled to a fluorogen-labelled particle, the immobilized reactant in the reaction zone 9 may be an antibody to the antigen, and the calibrator in the calibration zone 10 may be the analyte or an analyte analogue.

A predetermined amount of sample is added through the opening 3 in the housing part 1. All the necessary assay liquids, i.e. in this case sample liquid and buffer liquid, are then present in the device, the pull-out film 5, however, effectively preventing contact between the respective liquids and the membrane strip 6. The assay is then started by the operator removing the pull-out film 5 to thereby put the membrane strip 6 in simultaneous liquid receiving contact with the buffer pad 12 and the sample liquid in the sample well 3.

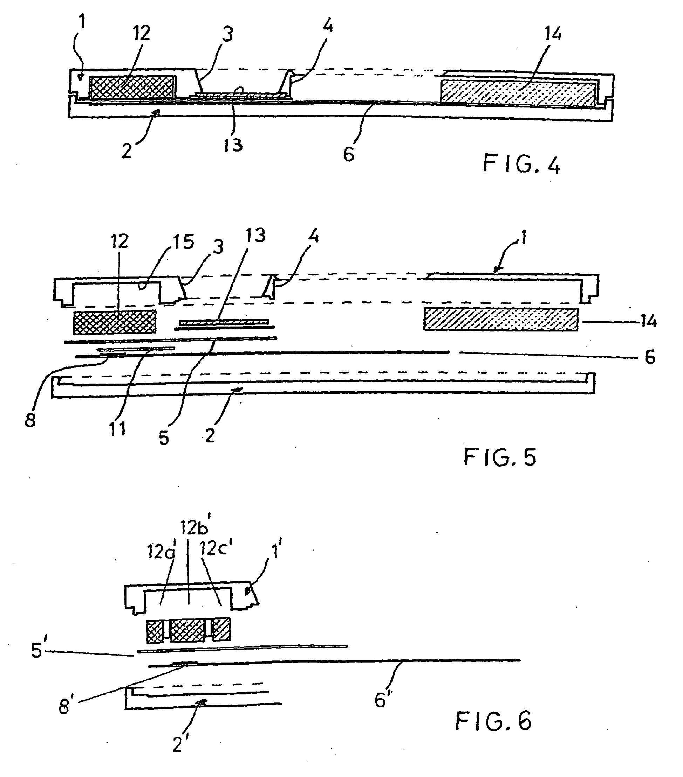

Buffer liquid from the pad 12 will now penetrate into the membrane strip 6 via the far upstream end part thereof which is in direct contact with the pad 12 (see Fig. 5) and be transported downstream the membrane strip 6 by capillary force. Simultaneously, sample liquid will penetrate into the membrane strip 6 and be transported in the downstream direction of the strip. There will thus be a flow of sample liquid directly followed by a flow pulse of buffer liquid. However, the detection reagent filter 8 and a major part of the buffer pad 12 are separated from the membrane strip 6 by the flow barrier film 11. Buffer liquid that has been transported into the membrane strip 6 will penetrate into and be transported through the filter 8 and bring the detection reagent deposited therein with it forming a detection reagent pulse. This detection reagent flow pulse will follow in sequence after the sample flow and the buffer flow pulse. Buffer that is transported in the membrane strip 6 after the detection reagent has been removed from the filter 8 will form a second buffer flow pulse following after the detection reagent flow pulse.

The above-mentioned different liquid flows will be transported along the membrane strip 6 in the indicated sequence, i.e. sample flow, first buffer flow, detection reagent flow, and second buffer flow, and will eventually reach the calibrator zone 10 and the reaction zone 9. In the reaction zone 9, analyte present in the sample will be captured by the reagent immobilized in the membrane. The analyte/capture reagent complex formed will be washed by the following first buffer flow, and the analyte-reagent complex will then react with detection reagent contained in the detection reagent flow to form a detectable detection reagent/capture reagent complex. The latter will finally be washed by the second buffer flow. In the calibration zone 10, the pre-determined amount of analyte therein will react with the detection reagent in the detection reagent flow to form a detectable detection reagent/analyte complex. The flow liquid from the buffer pad 12 will thus in sequence wash, dissolve detection reagent, and wash. By measuring, through the detection window formed by the opening 4 in the housing part 1, the signal intensity from the detection reagent captured in the reaction zone 9 and correlating it with that obtained in the calibration zone 10, the amount of analyte in the sample may be determined.

As apparent from the above, an assay with the described device is easy and convenient to perform and provides for simultaneous initiation of the different assay liquid flows. Thus, once the sample has been added to the sample well, the pull-out film may be removed. The liquid in the buffer pad and the sample will thereby be brought into contact with the membrane strip and the desired sequential transport of the different liquid flows will start.

Fig. 6 illustrates a variant embodiment of device according to the present invention. This embodiment differs from that in Figs. 2 to 5 in that (i) the single buffer pad 12 in the embodiment of Figs. 2 to 5 has been replaced by three different buffer pads 12'a, 12'b and 12'c, (ii) the flow barrier film 11 is omitted and the detection reagent filter, here designated 8', is placed below the central buffer pad 12'b. Also this embodiment will give the same sequence of liquid flows as that in Figs. 2 to 5 once the pull-out film 5 has been removed, i.e: sample flow, first wash flow, detection reagent flow, and second wash flow.

In another variation of device according to the present invention, the buffer pads 12'a and 12'b in Fig. 6 are combined to a single buffer pad, and the detection reagent filter 8' in Fig. 6 (with or without flow barrier film similar to the barrier film 11 in Fig. 5) is placed below the downstream part of the combined buffer pad. Also this embodiment will provide the same sequence of liquid flows upon removal of the pull-out film. The buffer 12'c will wash, and the combined buffer pad 12'a, 12'b will dissolve detection reagent and then wash.

Still another variation of device according to present invention has the buffer pad arrangement of Fig. 6 with three separate buffer pads 12'a, 12'b and 12'c but the detection reagent filter 8 i Figs. 2-5 is removed and the detection reagent is pre-deposited dissolved in the central buffer pad 12'b. Optionally, one or both of the two flanking buffer pads 12'a and 12'c may be omitted in this embodiment.

Other buffer pad arrangements as well as other variations and changes of the device which has been described above by way of example only, will be obvious to the skilled person.

In the reaction (or detection) zone described above, a reactant capable of specifically binding the analyte is immobilized (by covalent binding, via physical adsorption, via biospecific affinity, via immobilized particles to which the reactant is covalently bound, etc.). However, instead an agent capable of reacting with the reactant may be immobilized in the membrane, and the reactant may then be added together with the sample, or be pre-deposited in the membrane in an area or zone upstream of the reaction zone. Such an agent may be one member of a specific binding pair (sbp) and the reactant is then coupled or conjugated to the other member of the spb. Exemplary specific binding pairs include immunological binding pairs, such as antigen-antibody and hapten-antibody, biotin-avidin or -streptavidin, lectin-sugar, hormone-hormone receptor, nucleic acid duplex. For example, the reaction zone may have streptavidin immobilized therein and the capture reactant for the analyte may be biotinylated.

Similarly, the calibration zone may contain a binder for the calibrator substance rather than the calibrator substance *per se.* The binder is usually a member of a specific binding pair, such as one of those mentioned above, whereas the other member of the specific binding pair is coupled or conjugated to the calibrator substance, which may in turn be added with the sample or pre-deposited upstream of the calibrator zone. Streptavidin, for example, may be immobilized in the calibrator zone while the calibrator substance is biotinylated.

For further details on assay devices of the type contemplated herein, and particularly regarding flow matrixes, sequential assays, calibrator systems and detection reagents, it may be referred to our published international applications WO 99/36776, WO 99/36777 and WO 99/36780, for example.

Analytes to be determined using the present device are readily apparent to the skilled person. Usually, however, the analyte is a biospecific affinity reactant, e.g. an antibody or other protein, hapten, nucleic acid or polynucleotide, such as a DNA sequence. In the latter case the reaction zone may contain streptavidin and the DNA sequence to which the analyte sequence is to hybridize may be biotinylated.

The present device permits convenient pretreatment of the sample before starting the assay.

The present device may also be adapted for performing assays of the type described in our PCT application WO 9 980 402 where the flow matrix contains a chromatographic separation zone upstream of the reaction (detection) zone to separate sample components which would otherwise disturb or influence the determination of the analyte.

## Claims

1. A test device for conducting an assay for the determination of an analyte in a sample, said device comprising
a housing (1, 2) comprising
i) a flow matrix (6) in the form of a membrane strip of a bibulous material located within said housing, the material in the flow matrix (6) allowing liquid to be transported by capillary action, said flow matrix having at least one zone with immobilized capturing agent capable of directly or indirectly binding to the analyte,
ii) a sample well aperture (3) for sample liquid, and
iii) at least one liquid container (12) for liquid other than sample liquid,
**characterized in that**
the sample well aperture (3) for sample liquid and the liquid container(s) (12) for liquid other than sample liquid are arranged above the membrane strip (6), and **in that**
there is provided a separation element (5) to prevent contact between the test strip (6) and the bottom parts of the container(s) (12) and the well (3), respectively, said separation element (5) being manually removable so as to essentially simultaneously expose the top face of the test strip (6) to the bottom parts of the sample well (3) and the liquid container (12), respectively.

2. The test device according to claim 1, **characterized in that** the flow matrix (6) is flat and the liquid flow is lateral within said matrix.

3. The test device according to claim 2, **characterized in that** said liquid container (12) and well (3) are mounted adjacent to a face of said flow matrix (6), and the separation means comprise a flat liquid-tight element (5) sandwiched between the liquid containers (12) and the sample well (3), respectively, and the flow matrix (6).

4. The test device according to any one of claims 2 to 3, **characterized in that** the liquid-tight element (5) is at least partially removable from the housing (1, 2).

5. The test device according to claim 4, **characterized in that** the liquid-tight element (5) is a pull-out element, e.g. a pull-out sheet or film.

6. The test device according to any one of claims 1 to 5, **characterized in that** said at least one liquid container for liquid other than sample liquid comprise at least one container with flow liquid (12), such as a buffer solution.

7. The test device according to claim 6, **characterized in that** said liquid container or containers for flow liquid are in the form of an absorbent pad or sponge (12).

8. The test device according to any one of claims 1 to 7, **characterized in that** said at least one liquid container for liquid other than sample liquid comprise a container for an analytically detectable reagent.

9. The test device according to claim 8, **characterized in that** said liquid container for analytically detectable reagent is in the form of an absorbent pad or sponge.

10. The test device according to claim 8 or 9, **characterized in that** at least one liquid container for flow liquid is provided upstream and/or downstream of said container with analytically detectable reagent.

11. The test device according to any one of claims 1 to 7, **characterized in that** said flow matrix (6) comprises a zone having said analytically detectable reagent predeposited in the matrix or in an element (8) placed on the matrix.

12. The test device according to claim 11, **characterized in that** a first container for flow liquid is provided above and along said zone with analytically detectable reagent.

13. The test device according to claim 12, **characterized in that** at least one second container for flow liquid is provided upstream of said first container, and/or at least one third container is provided downstream of said first container.

14. The test device according to claim 11, **characterized in that that** a first container (12) for flow liquid extends both upstream of and at least partially above and along said zone with analytically detectable reagent.

15. The test device according to claim 11, **characterized in that** at least one second container for flow liquid is provided downstream of said first container.

16. The test device according to claim 14 or 15, **characterized in that** a barrier element (11) extends above said zone (8) with analytically detectable reagent to prevent direct contact between said first container (12) for flow liquid and the zone with analytically detectable reagent, when said separation means (5) is in said second position.

17. The test device according to any one of claims 1 to 16, **characterized in that** said capturing agent immobilized in the flow matrix (6) is a member of a specific binding pair and that the other member of the specific binding pair is part of or coupled to a reagent capable of binding the analyte.

18. The test device according to claim 17, **characterized in that** said specific binding pair is antigen-antibody, hapten-antibody, biotin-avidin, biotin-streptavidin or a nucleic acid duplex.

19. The test device according to any one of claims 8 to 18, **characterized in that** said analytically detectable reagent is labelled, such as by a nuorophore or a chromophore.

20. A method of performing an assay for determining an analyte in a sample, which method comprises flowing sample and assay liquids through a flow matrix to reach a reaction zone in said flow matrix in a predetermined sequence, **characterized in that** a device according to any one of claims 1 to 21 is used to carry out the method.

21. Use of the device according to any one of claims 1 to 18 for testing for an analyte in a sample indicating a disease selected from allergy, inflammation and autoimmune diseases.

22. The use according to claim 21, wherein the analyte is a specific immunoglobulin.

23. A kit for conducting an assay method, which kit comprises the device of any one of claims 1 to 19 in combination with other assay component(s).

## Patentansprüche

1. Testvorrichtung zur Durchführung eines Assays zur Bestimmung eines Analyten in einer Probe, wobei die Vorrichtung aufweist
ein Gehäuse (1,2), das aufweist
i) eine Fließmatrix (6) in Form eines sich innerhalb des Gehäuses befindlichen Membranstreifens aus einem saugfähigen Material, wobei es das Material in der Fließmatrix (6) der Flüssigkeit erlaubt, durch Kapillarwirkung transportiert zu werden, die Fließmatrix mindestens eine Zone mit einem immobilisierten Einfangmittel aufweist, das direkt oder indirekt an den Analyten binden kann,
ii) eine Probenmuldenöffnung (3) für Probenflüssigkeit und
iii) mindestens einen Flüssigkeitsbehälter (12) für eine von der Probenflüssigkeit verschiedene Flüssigkeit,
**dadurch gekennzeichnet, dass**
die Probenmuldenöffnung (3) für die Probenflüssigkeit und der/die Flüssigkeitsbehälter (12) für von der Probenflüssigkeit verschiedene Flüssigkeit oberhalb des Membranstreifens (6) angeordnet sind, und dass
ein Trennelement (5) vorgesehen ist, um den Kontakt zwischen dem Teststreifen (6) und den Bodenteilen des/der Behälter (12) bzw. der Mulde (3) zu verhindern, wobei das Trennelement (5) manuell so entfembar ist, dass es im wesentlichen gleichzeitig die Oberseite des Teststreifens (6) gegenüber den Bodenteilen der Probenmulde (3) bzw. den Flüssigkeitsbehälter (12) freilegt.

2. Testvorrichtung nach Anspruch 1, **dadurch gekennzeichnet, dass** die Fließmatrix (6) flach ist und die Flüssigkeitsströmung innerhalb der Matrix lateral ist.

3. Testvorrichtung nach Anspruch 2, **dadurch gekennzeichnet, dass** der Flüssigkeitsbehälter (12) und die Mulde (3) benachbart zu einer Fläche der Fließmatrix (6) befestigt sind, und das Trennmittel ein flaches flüssigkeitsdichtes Element (5) aufweist, das zwischen den Flüssigkeitsbehältern (12) bzw. der Probenmulde (3) und der Fließmatrix (6) sandwichartig angeordnet ist.

4. Testvorrichtung nach einem der Ansprüche 2 bis 3, **dadurch gekennzeichnet, dass** das flüssigkeitsdichte Element (5) zumindest teilweise aus dem Gehäuse (1, 2) entfernbar ist.

5. Testvorrichtung nach Anspruch 4, **dadurch gekennzeichnet, dass** das flüssigkeitsdichte Element (5) ein herausziehbares Element, z.B. eine herausziehbare Folie oder Film, ist.

6. Testvorrichtung nach einem der Ansprüche 1 bis 5, **dadurch gekennzeichnet, dass** der mindestens eine Flüssigkeitsbehälter für eine von der Probenflüssigkeit verschiedene Flüssigkeit mindestens einen Behälter mit Fließflüssigkeit (12), wie z.B. einer Pufferlösung, aufweist.

7. Testvorrichtung nach Anspruch 6, **dadurch gekennzeichnet, dass** der Flüssigkeitsbehälter oder die Flüssigkeitsbehälter für die Fließflüssigkeit in Form einer absorbierenden Auflage oder eines Schwammes (12) vorliegen.

8. Testvorrichtung nach einem der Ansprüche 1 bis 7, **dadurch gekennzeichnet, dass** der mindestens eine Flüssigkeitsbehälter für von der Probenflüssigkeit verschiedene Flüssigkeit einen Behälter für ein analytisch bestimmbares Reagens aufweist.

9. Testvorrichtung nach Anspruch 8, **dadurch gekennzeichnet, dass** der Flüssigkeitsbehälter für das analytisch bestimmbare Reagens in Form einer absorbierenden Auflage oder eines Schwammes vorliegt.

10. Testvorrichtung nach Anspruch 8 oder 9, **dadurch gekennzeichnet, dass** mindestens ein Flüssigkeitsbehälter für Fließflüssigkeit stromaufwärts und/oder stromabwärts von dem Behälter mit dem analytisch bestimmbaren Reagens vorgesehen ist.

11. Testvorrichtung nach einem der Ansprüche 1 bis 7, **dadurch gekennzeichnet, dass** die Fließmatrix (6) eine Zone aufweist, die das analytisch bestimmbare Reagens in der Matrix vorab enthalten oder in einem auf der Matrix vorhandenen Element (8) aufweist.

12. Testvorrichtung nach Anspruch 11, **dadurch gekennzeichnet, dass** ein erster Behälter für Fließflüssigkeit oberhalb und entlang der Zone mit dem analytisch bestimmbaren Reagens vorgesehen ist.

13. Testvorrichtung nach Anspruch 12, **dadurch gekennzeichnet, dass** mindestens ein zweiter Behälter für Fließflüssigkeit stromaufwärts vom ersten Behälter vorgesehen ist und/oder mindestens ein dritter Behälter stromabwärts vom ersten Behälter vorgesehen ist.

14. Testvorrichtung nach Anspruch 11, **dadurch gekennzeichnet, dass** sich der erste Behälter (12) für Fließflüssigkeit stromaufwärts von und zumindest teilweise oberhalb und entlang der Zone mit dem analytisch bestimmbaren Reagens erstreckt.

15. Testvorrichtung nach Anspruch 11, **dadurch gekennzeichnet, dass** mindestens ein zweiter Behälter für Fließflüssigkeit stromabwärts vom ersten Behälter vorgesehen ist.

16. Testvorrichtung nach Anspruch 14 oder 15, **dadurch gekennzeichnet, dass** sich ein Trennelement (11) oberhalb der Zone (8) mit analytisch bestimmbarem Reagens erstreckt, um einen direkten Kontakt zwischen dem ersten Behälter (12) für Fließflüssigkeit und der Zone mit dem analytisch bestimmbaren Reagens zu verhindern, wenn das Trennelement (5) in der zweiten Position ist.

17. Testvorrichtung nach einem der Ansprüche 1 bis 16, **dadurch gekennzeichnet, dass** das in der Fließmatrix (6) immobilisierte Einfangmittel ein Teil eines spezifischen Bindungspaares ist, und dass der andere Teil des spezifischen Bindungspaares Bestandteil des oder an das Reagens gebunden ist, das dazu fähig ist, den Analyten zu binden.

18. Testvorrichtung nach Anspruch 17, **dadurch gekennzeichnet, dass** das spezifische Bindungspaar Antigen-Antikörper, Hapten-Antikörper, Biotin-Avidin, Biotin-Streptavidin oder ein Nucleinsäure-Duplex ist.

19. Testvorrichtung nach einem der Ansprüche 8 bis 18, **dadurch gekennzeichnet, dass** das analytisch bestimmbare Reagens markiert ist, wie z.B. durch ein Fluorophor oder ein Chromophor.

20. Methode zur Durchführung eines Assays zur Bestimmung eines Analyten in einer Probe, wobei die Methode das Durchfließen lassen von Probe und Assayflüssigkeiten durch eine Fließmatrix umfasst, um eine Reaktionszone in dieser Fließmatrix in einer bestimmten Sequenz zu erreichen, **dadurch gekennzeichnet, dass** zur Durchführung der Methode eine Vorrichtung nach einem der Ansprüche 1 bis 19 verwendet wird.

21. Verwendung einer Vorrichtung nach einem der Ansprüche 1 bis 18 zum Testen eines Analyten in einer Probe, der eine Erkrankung anzeigt, ausgewählt aus Allergie, Entzündung und Autoimmun-Erkrankungen.

22. Verwendung nach Anspruch 21, worin der Analyt ein spezifisches Immunoglobulin ist.

23. Kit zur Durchführung einer Assay-Methode, wobei der Kit die Vorrichtung nach einem der Ansprüche 1 bis 19 in Kombination mit einer anderen Assay-Komponente(n) aufweist.

## Revendications

1. Dispositif d'essai servant à effectuer un dosage pour déterminer une substance à analyser dans un échantillon, ledit dispositif comprenant un logement (1, 2) comprenant
i) une matrice d'écoulement (6) ayant la forme d'une bande de membrane constituée d'un matériau absorbant située à l'intérieur dudit logement, le matériau dans la matrice d'écoulement (6) permettant au liquide d'être transporté par action capillaire, ladite matrice d'écoulement ayant au moins une zone dotée d'un agent de capture immobilisé capable de se lier directement ou indirectement à la substance à analyser,
ii) un orifice (3) de puits à échantillons pour le liquide de l'échantillon et
iii) au moins un récipient à liquide (12) pour un liquide autre que le liquide d'échantillon,
**caractérisé en ce que**
l'orifice du puits à échantillons (3) servant au liquide d'échantillon et le(s) récipient(s) à liquide (12) pour le liquide autre que le liquide d'échantillon sont placés au-dessus de la bande de membrane (6) et **en ce qu'** il y a un élément de séparation (5) prévu pour empêcher le contact entre la bande d'essai (6) et les parties inférieures du (des) récipient(s) (12) et le puits (3), respectivement, ledit élément de séparation (5) étant amovible manuellement de manière à essentiellement exposer simultanément la face supérieure de la bande d'essai (6) aux parties inférieures du puits à échantillons (3) et au récipient à liquide (12), respectivement.

2. Dispositif d'essai selon la revendication 1, **caractérisé en ce que** la matrice d'écoulement (6) est plate et que l'écoulement liquide est latéral à l'intérieur de ladite matrice.

3. Dispositif d'essai selon la revendication 2, **caractérisé en ce que** ledit récipient à liquide (12) et le puits (3) sont montés adjacents à une face de ladite matrice d'écoulement (6) et que les moyens de séparation comprennent un élément plat étanche au liquide (5), intercalé entre les récipients à liquide (12) et le puits à échantillons (3), respectivement, et la matrice d'écoulement (6).

4. Dispositif d'essai selon l'une quelconque des revendications 2 à 3, **caractérisé en ce que** l'élément étanche au liquide (5) peut au moins en partie être retiré du logement (1, 2).

5. Dispositif d'essai selon la revendication 4, **caractérisé en ce que** l'élément étanche au liquide (5) est un élément pouvant être arraché, par exemple une feuille ou un film pouvant être arraché.

6. Dispositif d'essai selon l'une quelconque des revendications 1 à 5, **caractérisé en ce que** ledit au moins un récipient à liquide pour le liquide autre que le liquide d'échantillon
comprend au moins un récipient à liquide d'écoulement (12), tel qu'une solution tampon.

7. Dispositif d'essai selon la revendication 6, **caractérisé en ce que** ledit récipient à liquide ou lesdits récipients à liquide d'écoulement sont sous la forme d'un tampon absorbant ou d'une éponge (12).

8. Dispositif d'essai selon l'une quelconque des revendications 1 à 7, **caractérisé en ce que** ledit au moins un récipient à liquide pour liquide autre que le liquide d'échantillon comprend un récipient pour un réactif détectable par analyse.

9. Dispositif d'essai selon la revendication 8, **caractérisé en ce que** ledit récipient à liquide pour réactif détectable par analyse est sous la forme d'un tampon absorbant ou d'une éponge.

10. Dispositif d'essai selon la revendication 8 ou 9, **caractérisé en ce qu'**au moins un récipient à liquide pour liquide d'écoulement est prévu en amont et/ou en aval dudit récipient ayant un réactif détectable par analyse.

11. Dispositif d'essai selon l'une quelconque des revendications 1 à 7, **caractérisé en ce que** ladite matrice d'écoulement (6) comprend une zone ayant ledit réactif détectable par analyse déposé d'avance dans la matrice ou dans un élément (8) placé sur la matrice.

12. Dispositif d'essai selon la revendication 11, **caractérisé en ce qu'**un premier récipient pour liquide d'écoulement est prévu au-dessus et le long de ladite zone ayant un réactif détectable par analyse.

13. Dispositif d'essai selon la revendication 12, **caractérisé en ce qu'**au moins un deuxième récipient à liquide d'écoulement est prévu en amont dudit premier récipient et/ou qu'au moins un troisième récipient est prévu en aval dudit premier récipient.

14. Dispositif d'essai selon la revendication 11, **caractérisé en ce qu'**un premier récipient (12) à liquide d'écoulement s'étend à la fois en amont de et au moins en partie au-dessus et le long de ladite zone ayant un réactif détectable par analyse.

15. Dispositif d'essai selon la revendication 11, **caractérisé en ce qu'**au moins un deuxième récipient à liquide d'écoulement est prévu en aval dudit premier récipient.

16. Dispositif d'essai selon la revendication 14 ou 15, **caractérisé en ce qu'**un élément de barrière (11) s'étend au-dessus de ladite zone (8) ayant un réactif détectable par analyse pour empêcher un contact direct entre ledit premier récipient (12) à liquide d'écoulement et la zone ayant un réactif détectable par analyse, quand ledit moyen de séparation (5) est dans ladite deuxième position.

17. Dispositif d'essai selon l'une quelconque des revendications 1 à 16, **caractérisé en ce que** ledit agent de capture immobilisé dans la matrice d'écoulement (6) est un élément d'une paire de liaison spécifique et que l'autre élément de la paire de liaison spécifique fait partie de ou est couplé avec un réactif capable de lier la substance à analyser.

18. Dispositif d'essai selon la revendication 17, **caractérisé en ce que** ladite paire de liaison spécifique est un antigène-anticorps, un haptène-anticorps, une biotine-avidine, une biotine-streptavidine ou un duplex d'acide nucléique.

19. Dispositif d'essai selon l'une quelconque des revendications 8 à 18, **caractérisé en ce que** ledit réactif détectable par analyse est marqué, comme par un fluorophore ou un chromophore.

20. Procédé d'exécution d'un dosage pour déterminer une substance à analyser dans un échantillon, lequel procédé comprend la mise en écoulement de liquides d'échantillon et de dosage à travers une matrice d'écoulement pour atteindre une zone de réaction dans ladite matrice d'écoulement suivant une séquence prédéterminée,
**caractérisé en ce qu'**un dispositif selon l'une quelconque des revendications 1 à 21 est utilisé pour mettre à exécution ce procédé.

21. Emploi d'un dispositif selon l'une quelconque des revendications 1 à 18 pour rechercher s'il y a une substance à analyser dans un échantillon qui indiquerait une maladie choisie parmi une allergie, une inflammation et des maladies auto-immunes.

22. Emploi selon la revendication 21, dans laquelle la substance à analyser est une immunoglobuline spécifique.

23. Trousse (« kit ») servant à exécuter un procédé de dosage, la trousse (« kit ») comprenant le dispositif selon l'une quelconque des revendications 1 à 19 combiné avec un ou d'autres composants de dosage.
